# EUROPÄISCHE PATENTSCHRIFT

(11) **EP 1 667 591 B1**
(45) Veröffentlichungstag und Bekanntmachung des Hinweises auf die Patenterteilung: **01.07.2009**
(21) Anmeldenummer: 03818769.6
(22) Anmeldetag: 29.09.2003
(51) Int. Cl.: A61B 17/70

(54) **VORRICHTUNG ZUR ELASTISCHEN STABILISIERUNG VON WIRBELKÖRPERN**
DEVICE FOR ELASTICALLY STABILISING VERTEBRAL BODIES
DISPOSITIF DE STABILISATION ELASTIQUE DE CORPS VERTEBRAUX

(43) Veröffentlichungstag der Anmeldung: 14.06.2006
(73) Patentinhaber: Synthes GmbH, 4436 Oberdorf (CH)
(72) Erfinder: HARTMANN, Stephan, CH-4500 Solothurn (CH); STUDER, Armin, CH-4513 Langendorf (CH)
(74) Vertreter: Lusuardi, Werther
(86) Internationale Anmeldenummer: PCT/CH2003/000647
(87) Internationale Veröffentlichungsnummer: WO 2005/030066

(56) Entgegenhaltungen:
- EP-A- 0 667 127
- GB-A- 2 382 304
- US-B1- 6 206 882
- US-B1- 6 267 764
- US-B1- 6 440 169

## Beschreibung

Die Erfindung bezieht sich auf eine Vorrichtung zur elastischen Stabilisierung von Wirbelkörpern, gemäss dem Oberbegriff des Patentanspruchs 1.

Bei Behandlungen von beschädigten oder tumorbehafteten Wirbelkörpern werden üblicherweise starre, mittels Knochenverankerungsmitteln, beispielsweise Pedikelschrauben oder Pedikelhaken an den Wirbelkörpern verankerte Längsträger eingesetzt. Auf diese Weise lässt sich eine Bewegung der stabilisierten Wirbelkörper relativ zueinander verhindern, so dass die Fusion der benachbarten Wirbelkörper gefördert wird.

Aus der FR-A-2 799 949 BENAZZA ist eine Wirbelsäulenfixations-Vorrichtung bekannt, welche aus einer Anzahl tulpenförmiger Pedikelschrauben besteht, welche statt des üblichen starren Längsträger mit einzelnen Spiralfederelementen untereinander verbunden sind. Die Länge der Spiralfedern ist zwar einstellbar, man erreicht aber damit nur eine Änderung der Vorspannkraft der Spiralfederelemente.

Aus der EP-A-0 516 567 NAVAS ist eine weitere Wirbelsäulenfixations-Vorrichtung bekannt, welche aus einer Anzahl tulpenförmiger Pedikelschrauben besteht, welche statt des üblichen starren Längsträger mit einzelnen Dämpfungselementen untereinander verbunden sind. Nachteilig an diesen Dämpfungselementen ist deren Herstellung, so dass das Dämpfungselement eine lineare Federkennlinie aufweist.

Weitere Wirbelsäulenfixations-Vorrichtungen mit parallel zur Wirbelsäulenlängsachse zwischen je zwei Pedikelschrauben angeordneten elastischen Verbindungsteilen sind aus der GB 2 382 304 DILIP, der EP 0 667 127 ACROMED und der US 6 267 764 ELBERG bekannt.

Aus der US 6,206,882 COHEN ist eine weitere Wirbelsäulenfixations-Vorrichtung bekannt, welche eine mit mehreren Schlitzen ausgestattete Platte umfasst.

Eine weitere Wirbelsäulenfixations-Vorrichtung mit zwei Verankerungselementen und dazwischen angeordneten elastischen Mitteln ist aus der US 6,440,169 ELBERG bekannt.

Eine weitere Wirbelsäulenfixations-Vorrichtung mit einer Anzahl Pedikelschrauben und parallel zur Wirbelsäulenlängsachse zwischen je zwei Pedikelschrauben angeordneten elastischen Verbindungsteilen ist aus der EP 0 677 277 MOREAU bekannt. Diese bekannte Vorrichtung umfasst elastische Verbindungsteile mit progressiven Federkennlinien. Die elastischen Mittel umfassen eine Schraubenfeder, deren Zentralbohrung mit einem viskoelastischen Material gefüllt ist. Nachteilig an dieser bekannten Vorrichtung ist, dass durch die Ausgestaltung der elastischen Mittel mit zwei eine unterschiedliche Federrate aufweisenden Elementen ein aufwendiger Herstellungsprozess der elastischen Mittel notwendig wird.

Hier will die Erfindung Abhilfe schaffen. Der Erfindung liegt die Aufgabe zugrunde, eine Vorrichtung zur Stabilisierung von Wirbelkörpern zu schaffen, welche nur ein aus einem metallischen Material hergestelltes Federelement mit einer bei Druckbelastung progressiven Federkennlinie umfasst.

Die Erfindung löst die gestellte Aufgabe mit einer Vorrichtung zur elastischen Stabilisierung von Wirbelkörpern, welche die Merkmale des Anspruchs 1 aufweist.

Die durch die Erfindung erreichten Vorteile sind im wesentlichen darin zu sehen, dass dank der efindungsgemässen Vorrichtung
- bei kleinen Druckkräften eine ausreichend grosse elastische Nachgiebigkeit und Dämpfung gewährleistet ist. Dadurch wird eine ausreichende Bewegungsfreiheit der Wirbelsäule in diesem Bereich ermöglicht;
- bei hohen Druckbelastungen oder Stossbelastungen keine grossen Federwege zur Aufnahme der Druckkraft notwendig sind, so dass eine Überlastung der posterioren Elemente verhindert werden kann;
- mit einem einzigen aus einem biokompatiblen metallischen Werkstoff, beispielsweise aus Titan hergestellten Federelement eine stufenlos progressive Federkennline erreichbar ist.

Die Federkennlinie der elastischen Mittel weist einen stufenlos progressiven Verlauf im Kraft-Weg-Diagramm auf. Dabei ist das elastische Mittel vorzugsweise als Schraubenfeder mit einer variierenden Steigung der Federwindungen ausgebildet.

Weitere Ausgestaltungen einer Schraubenfeder mit progressiver Federrate sind beispielsweise durch
- Herstellung einer Schraubenfeder möglich, welche parallel zur Längsachse gemessen mindestens zwischen zwei benachbarten Federwindung eine verschiedene Schlitzbreite x aufweist; oder
- Herstellung einer Schraubenfeder aus einem Federmaterial, dessen zur Längsachse orthogonale Querschnittsfläche parallel zur Längsachse gemessen zwischen mindestens zwei benachbarten Federwindung eine verschiedene Höhe h aufweist.

In einer nicht erfindungsgemäßen Ausführungsform sind die elastischen Mittel als ebene, mäanderartige Feder mit mehreren entlang Längsachse hintereinander angeordneten Federwindungen ausgebildet, wobei jede Federwindung eine Wendeschleife mit einer Biegeachse aufweist.

Je nach Ausführungsform können die Abstände L zwischen den Biegeachsen und der Längsachse beiderseits der Längsachse konstant sein, oder die Federwindungen können so ausgeführt sein, dass der Abstand L1 zwischen der mindestens einen, auf der linken Seite der Längsachse angeordneten Biegeachse und der Längsachse gegenüber dem Abstand L2 zwischen der mindestens einen, auf der rechten Seite der Längsachse angeordneten Biegeachse und der Längsachse verschieden ist.

In wiederum einer anderen Ausführungsform umfasst jede Federwindung Arretiermittel, welche den zulässigen Federweg der entsprechenden Federwindung begrenzen. Vorzugsweise umfassen die Arretiermittel pro Federwindung mindestens einen Nocken, welcher den Federweg s der entsprechenden Federwindung begrenzt.

Durch die Ausgestaltung der Nocken kann der durch den mindestens einen Nocken begrenzte Federweg s für mindestens zwei Federwindungen verschieden sein.

Die mäanderförmige Feder kann n Federwindungen aufweisen, wobei jede Federwindung eine verschiedene Federrate i aufweist, d.h. beispielsweise i₁ < iⱼ < in ist.

In einer weiteren Ausführungsform ist die Wandstärke des Federmaterials von mindestens zwei Wendeschleifen verschieden, so dass für diese zwei Federwindungen die Federraten verschieden sind.

In wiederum einer weiteren Ausführungsform umfassen die elastischen Mittel an ihren axial aussenstehenden Enden Verbindungsteile, welche zur Fixation der elastischen Mittel an Knochenverankerungsmitteln geeignet sind. Beispielsweise kann eines der zwei Verbindungsteile als zur Längsachse koaxialer Stab ausgestaltet sein, während das zweite Verbindungsteil als Hülse mit einer zur Längsachse koaxialen Zentralbohrung zur Aufnahme eines stabförmigen Längsträgers ausgebildet sein kann. Andererseits wäre auch mindestens ein Verbindungsteil möglich, welches mittels eines Gelenkverbindung mit einem beispielsweise stabförmigen Längsträger verbindbar wäre.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Ausführungsform der erfindungsgemässen Vorrichtung zur Stabilisierung mehrerer Wirbelkörper;
Fig. 2 einen Längsschnitt durch die elastischen Mittel einer Ausführungsform der erfindungsgemässen Vorrichtung;
Fig. 3 eine Ansicht der elastischen Mittel einer nicht erfindungsgemäßen Ausführungsform der Vorrichtung;
Fig. 4a und 4b die Federkennlinien im Kraft-Weg Diagramm der elastischen Mittel der in Fig. 2 bzw. Fig. 3 dargestellten Ausführungsformen der Vorrichtung;

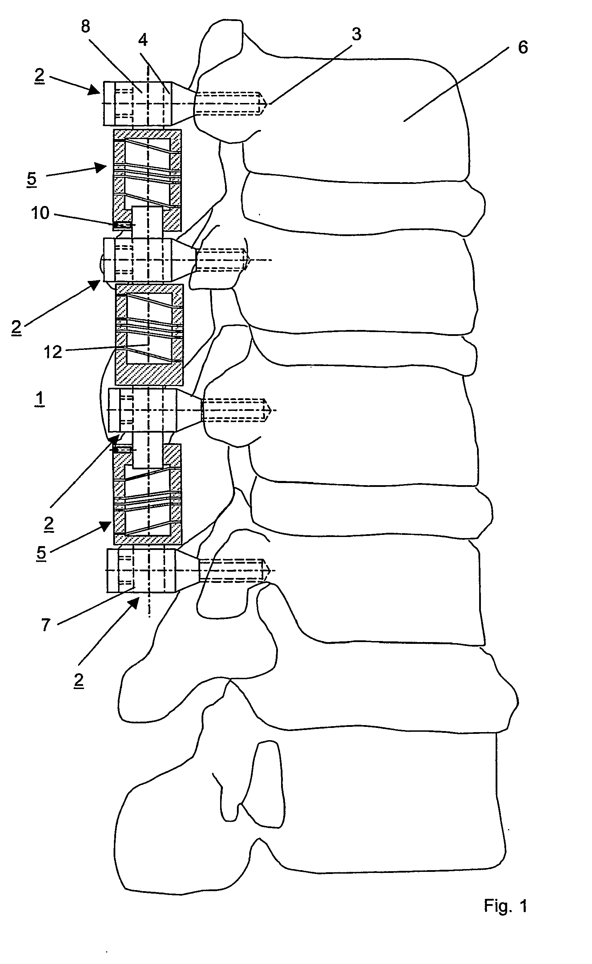

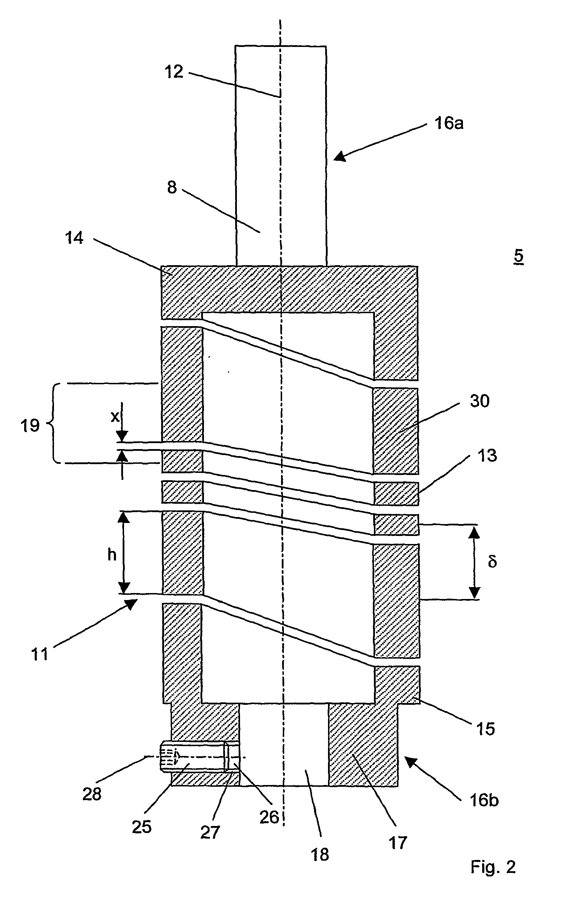

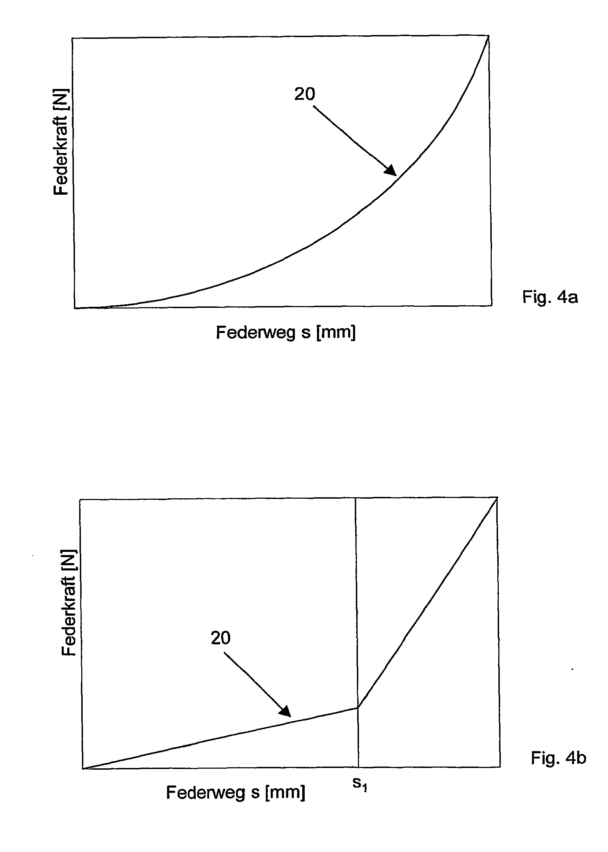

In Fig.1 ist eine Ausführungsform einer Vorrichtung 1 zur Stabilisierung benachbarter Wirbelkörper 6 dargestellt. Mehrere beispielsweise als Pedikelschrauben ausgebildete Knochenverankerungsmittel 2 sind so in die Pedikel der zu verbindenden Wirbelkörper 6 eingeschraubt, dass ihre Zentralachsen 3 quer zur Wirbelsäulenlängsachse angeordnet sind. Die Kopfsegmente 4 der Knochenverankerungsmittel 2 sind koaxial zu den Zentralachsen 3 der Knochenverankerungsmittel 2 angeordnet und weisen quer zur Zentralachse 3 verlaufende Kanäle 7 auf. In diesen Kanälen 7 sind die stabförmigen Verbindungsteile 8 der elastischen Mittel 5 einführbar, so dass die hier als Schraubenfedern ausgestalteten elastischen Mittel 5 relativ zu den Kanälen 7 und parallel zur Wirbelsäulenlängsachse verschiebbar sind, bevor sie mittels in den Kopfsegmenten 4 angeordneten Schrauben 10 relativ zu den Knochenverankerungsmitteln 2 fixiert werden. Die elastischen Mittel 5 sind bezüglich ihrer Längsachse 12 axial und auf Biegung elastisch deformierbar, so dass die Federwege s (Fig. 4) der Knochenverankerungsmittel 2 ebenfalls parallel zur Längsachse 12 sind. Die zwei axial aussenstehenden elastischen Mittel 5 sind mit Verbindungsteilen 16a;16b gemäss der in Fig. 2 dargestellten Ausführungsform ausgestaltet, während das mittlere elastische Mittel 5 zwei stabförmige, koaxiale Verbindungsteile 16a (Fig. 2) umfasst.

In Fig. 2 ist eine Ausführung der elastischen Mittel 5 als Schraubenfeder 11 dargestellt, deren Federmaterial eine zur Längsachse 12 parallele rechteckige Querschnittsfläche 13 aufweist, wobei die Querschnittsfläche 13 parallel zur Längsachse 12 gemessen eine variierende Höhe h aufweist. Durch die gegen die Enden 14;15 der Schraubenfeder 11 zunehmende Höhe h der Querschnittsfläche 13 des Federmaterials ergibt sich eine ebenfalls gegen die Enden 14;15 der Schraubenfeder 11 zunehmende Steigung 8 der Federwindungen. In der hier dargestellten Ausführungsform bleibt die Schlitzbreite x auf der gesamten Länge der Schraubenfeder 11 konstant. Der zur Längsachse 12 orthogonalen Querschnitt ist kreisringförmig ausgestaltet und die Schranbenfeder weist an ihrer beiden die Längsachse 12 schneidenden Enden 14;15 je ein Verbindungsmittel 16 auf, welches zur Fixierung der Schraubenfeder 11 an einem Knochenverankerungsmittel 2 (Fig. 1) dient. Das erste, am ersten Ende 14 der Schraubenfeder 11 angeordnete Verbindungsmittel 16a ist als zur Längsachse 12 koaxialer Stab 8 ausgebildet, während das zweite, am zweiten Ende 15 der Schraubenfeder 11 angeordnete Verbindungsmittel 16b als Hülse 17 ausgestaltet ist und eine zur Längsachse 12 koaxiale Zentralbohrung 18 umfasst. Beide Verbindungsmittel 16 sind fest mit der Schraubenfeder 11 verbunden. Durch die variable Steigung 8 der Federwindungen 19 der Schraubenfeder 11 wird eine progressive Federkennlinie erreicht. Zur Fixation eines stabförmigen Teils in der Zentralbohrung 18 ist eine Feststellschraube 25 vorgesehen, welche in eine ein komplementäres Innengewinde 27 aufweisende Bohrung 26 mit quer zur Längsachse 12 stehender Bohrungsachse 28 schraubbar und mit ihrem vorderen Ende gegen eine in die Zentralbohrung 18 eingeführten Stab (nicht gezeichnet) pressbar ist.

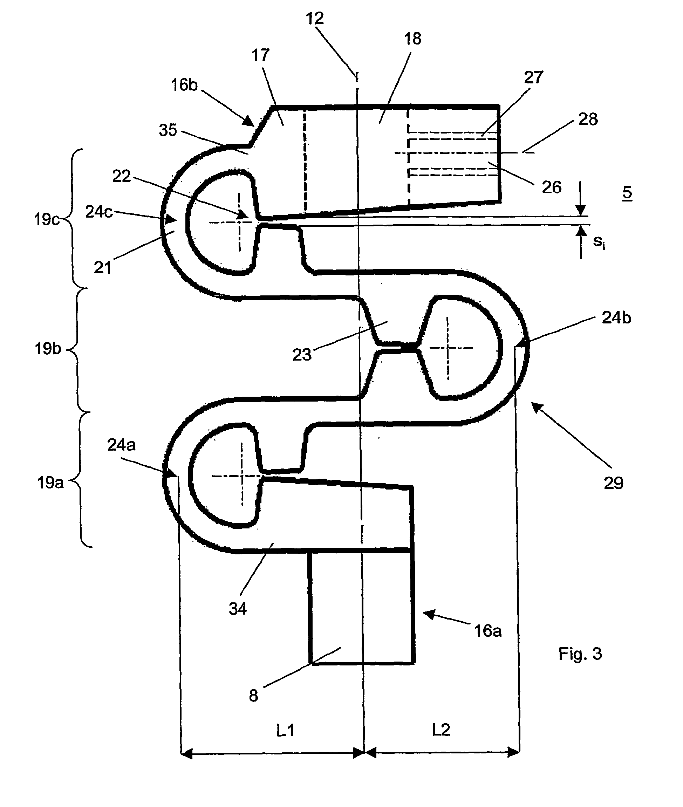

Fig. 3 zeigt ein nicht erfindungsgemäße Ausführungsform der elastischen Mittel 5, welche eine Feder 29 mit mehreren in einer Ebene liegenden Federwindungen 19 umfasst. Jede der drei Windung 19a;19b;19c weist eine auf Biegung beanspruchte Wendeschleife 21 und Arretiermittel 22 auf. Die Arretiermittel 22 bestehen hier aus je zwei Nocken 23, welche nach Erreichen des pro Windung 19 zulässigen Federweges aufeinander zu Anlage kommen und ein weiteres Einfedern der betreffenden Windung 19 verhindern. Die Wendeschleifen 21 der drei Federwindungen 19a;19b;19c weisen je eine Biegeachse 24a;24b;24c auf, wobei die Länge L1 der links der Längsachse 12 bezüglich der Biegung wirksamen Hebelarme grösser als die Länge L2 des rechts der Längsachse 12 wirksamen Hebelarmes ausgestaltet ist. Anstelle der verschieden langen Hebelarmen könnten auch die durch die Arretiermittel 22 zulässigen Federwege sᵢ pro Federwindung 19 verschieden sein. Analog zur in Fig. 2 dargestellten Ausführungsform weist die Feder 29 an ihren beiden die Längsachse 12 schneidenden Enden 34;35 je ein Verbindungsmittel 16 auf, welches zur Fixierung der elastischen Mittel 5 an einem Knochenverankerungsmittel 2 (Fig. 1) geeignet ist. Das erste, am ersten Ende 34 der Feder 29 angeordnete Verbindungsmittel 16a ist als zur Längsachse 12 koaxialer Stab 8 ausgebildet, während das zweite, am zweiten Ende 35 der Feder 29 angeordnete Verbindungsmittel 16b als Hülse 17 ausgestaltet ist und eine zur Längsachse 12 koaxiale Zentralbohrung 18 umfasst. Beide Verbindungsmittel 16 sind fest mit der Feder 29 verbunden. Zur Fixation eines stabförmigen Teils in der Zentralbohrung 18 ist eine Feststellschraube (nicht gezeichnet) vorgesehen, welche in eine ein komplementäres Innengewinde 27 aufweisende Bohrung 26 mit quer zur Längsachse 12 stehender Bohrungsachse 28 schraubbar und mit ihrem vorderen Ende gegen eine in die Zentralbohrung 18 eingeführten Stab (nicht gezeichnet) pressbar ist.

In Fig. 4a ist die Federkennlinie 20 für die in Fig. 2 dargestellte Ausführungsformen der elastischen Mittel 5 im Kraft-Weg Diagramm dargestellt. Die Federkennlinie 20 ist über dem gesamten Federweg progressiv. Eine solche Federkennlinie 20 ist beispielsweise durch die Ausgestaltung der elastischen Mittel 5 als Schraubenfeder 11 mit entlang der Längsachse 12 kontinuierlich variierender Steigung δ erreichbar.

Die in Fig. b dargestellte Federkennlinie 20 lässt sich durch eine Ausführung der elastischen Mittel 5 als mäanderförmige Feder 29 realisieren. Die Federkennlinie 20 gemäss Fig. 4b ist unstetig und weist bei einem Federweg s = s₁ einen Knickpunkt auf, welcher beispielsweise durch das Schliessen der Arretiermittel 22 einer Federwindung 19 realisierbar ist.

## Patentansprüche

1. Vorrichtung (1) zur elastischen Stabilisierung von Wirbelkörpern mit
A) mindestens zwei Knochenverankerungsmitteln (2), welche je eine Zentralachse (3) und ein Kopfsegment (4) aufweisen; und
B) elastischen Mitteln (5), welche eine Längsachse (12) aufweisen und derart mit den Kopfsegmenten (4) zweier benachbarter Knochenverankerungsmittel (2) verbindbar sind, dass die Längsachse (12) quer zu den Zentralachsen (3) steht, wobei C) die elastischen Mittel (5) bei Druckbelastung eine progressive Federkennlinie (20) aufweisen, aus einem metallischen Werkstoff hergestellt sind und nur ein Federelement umfassen; wobei
D) die elastischen Mittel (5) Federwindungen (19) umfassen, wovon mindestens zwei Federwindungen (19) mindestens eine voneinander verschiedene geometrische Abmessung aufweisen,
**dadurch gekennzeichnet, dass**
E) die Federkennlinie (20) der elastischen Mittel (5) einen stufenlos progressiven Verlauf im Kraft-Weg-Diagramm aufweist.

2. Vorrichtung (1) nach Anspruch 1, **dadurch gekennzeichnet, dass** die elastischen Mittel (5) als Schraubenfeder (11) mit einer variierenden Steigung δ der Federwindungen (19) ausgestaltet sind.

3. Vorrichtung (1) nach Anspruch 2, **dadurch gekennzeichnet, dass** die Schraubenfeder (11) parallel zur Längsachse (12) gemessen mindestens zwischen zwei benachbarten Federwindung (19) eine verschiedene Schlitzbreite x aufweist.

4. Vorrichtung (1) nach Anspruch 2 oder 3, **dadurch gekennzeichnet, dass** die Schraubenfeder (11) aus einem Federmaterial gefertigt ist, dessen zur Längsachse (12) parallele Querschnittsfläche (13) parallel zur Längsachse (12) gemessen zwischen mindestens zwei benachbarten Federwindung (19) eine verschiedene Höhe h aufweist.

5. Vorrichtung (1) nach einem der Ansprüche 1 bis 4, **dadurch gekennzeichnet, dass** die elastischen Mittel (5) an ihren Enden (3;4) Verbindungsteile (16) umfassen, welche zur Fixation der elastischen Mittel (5) an Knochenverankerungsmitteln (2) geeignet sind.

6. Vorrichtung (1) nach Anspruch 5, **dadurch gekennzeichnet, dass** mindestens ein Verbindungsteil (16a) ein zur Längsachse (25) koaxialer Stab (8) ist.

7. Vorrichtung (1) nach Anspruch 5 oder 6, **dadurch gekennzeichnet, dass** mindestens ein Verbindungsteil (16b) eine Hülse (17) mit einer zur Längsachse (12) koaxialen Zentralbohrung (18) ist.

8. Vorrichtung (1) nach einem der Ansprüche 5 bis 7, **dadurch gekennzeichnet, dass** mindestens ein Verbindungsteil (16) eine Gelenkverbindung umfasst.

## Claims

1. A device (1) for the elastic stabilisation of bodies of the vertebra with
A) at least two bone anchoring means (2), each of them having a central axis (3) and a head segment (4), and
B) elastic means (5), which have a longitudinal axis (12) and can be joined with the head segments (4) of two adjacent bone anchoring means (2) in such a manner, that the longitudinal axis (12) extends transversely to the central axes (3), while
C) the elastic means (5) have a progressive spring characteristics (20) under compressive load, is manufactured from a metallic material and comprises one spring element only; wherein
D) the elastic means (5) comprise spring coils (19), of which at least two spring coils (19) have at least one geometric dimension which is different,
**characterised in that**
E) the spring characteristics (20) of the elastic means (5) has a continuously progressive force-travel diagram.

2. A device (1) according to claim 1, **characterised in that** the elastic means (5) is constructed as a helical spring (11) with a varying pitch δ of the spring coils (19).

3. A device (1) according to claim 2, **characterised in that** measured parallel to the longitudinal axis (12) the helical spring (11) has a slot with a different width x at least between two adjacent spring coils (10).

4. A device (1) according to claim 2 or 3, **characterised in that** the helical spring (11) is manufactured from a spring material, the cross-sectional area (13) of which parallel to the longitudinal axis (12) and measured parallel to the longitudinal axis have different heights h at least between two adjacent spring coils (19).

5. A device (1) according to any one of claims 1 to 4, **characterised in that** the elastic means (5) comprise at their ends (3, 4) connecting parts (16), which are suitable for the fixing of the elastic means (5) on the bone anchoring means (2).

6. A device (1) according to claim 5, **characterised in that** at least one connecting part (16a) is a rod (8) that is coaxial with the longitudinal axis (12).

7. A device (1) according to claim 5 or 6, **characterised in that** at least one connecting part (16b) is a sleeve (17) with a central bore (18) that is coaxial with the longitudinal axis (12).

8. A device (1) according to any one of claims 5 to 7, **characterised in that** at least one connecting part (16) comprises a hinged joint.

## Revendications

1. Dispositif (1) pour la stabilisation élastique de corps vertébraux, ledit dispositif comportant :
A) au moins deux moyens (2) d'ancrage à un os qui possèdent chacun un axe central (3) et un segment de tête (4) ; et
B) des moyens élastiques (5) qui possèdent un axe longitudinal (12) et qui peuvent être reliés aux segments de tête (4) de deux moyens adjacents (2) d'ancrage à un os de telle sorte que l'axe longitudinal (12) est transversal aux axes centraux (3),
C) les moyens élastiques (5) présentant en cas de contrainte de compression une caractéristique de ressort progressive (20), étant fabriqués à partir d'un matériau métallique et ne comportant qu'un élément de ressort ; D) les moyens élastiques (5) comportant des spires de ressort (19) dont au moins deux spires de ressort (19) ont au moins une dimension géométrique variable d'une spire à l'autre,
**caractérisé en ce que**
E) la caractéristique de ressort (20) des moyens élastiques (5) a une allure progressive continue dans la courbe représentative Force/Chemin.

2. Dispositif (1) selon la revendication 1, **caractérisé en ce que** les moyens élastiques (5) sont conformés en ressort hélicoïdal (11) dont les spires de ressort (19) ont un pas δ variable.

3. Dispositif (1) selon la revendication 2, **caractérisé en ce que** le ressort hélicoïdal (11) a une largeur de fente x variable, mesurée parallèlement à l'axe longitudinal (12), au moins entre deux spires de ressort adjacentes (19).

4. Dispositif (1) selon la revendication 2 ou 3, **caractérisé en ce que** le ressort hélicoïdal (11) est fabriqué à partir d'un matériau pour ressort dont la section (13), parallèle à l'axe longitudinal (12), a une hauteur variable h, mesurée parallèlement à l'axe longitudinal (12), entre au moins deux spires de ressort adjacentes (19).

5. Dispositif (1) selon l'une des revendications 1 à 4, **caractérisé en ce que** les moyens élastiques (5) possèdent à leurs extrémités (3 ; 4) des pièces de liaison (16) qui sont appropriées pour fixer les moyens élastiques (5) à des moyens (2) d'ancrage à un os.

6. Dispositif (1) selon la revendication 5, **caractérisé en ce qu'**au moins une pièce de liaison (16a) est une barre (8) coaxiale à l'axe longitudinal (25).

7. Dispositif (1) selon la revendication 5 ou 6, **caractérisé en ce qu'**au moins une pièce de liaison (16b) est un manchon (17) doté d'un perçage central (18) coaxial à l'axe longitudinal (12).

8. Dispositif (1) selon l'une des revendications 5 à 7, **caractérisé en ce qu'**au moins une pièce de liaison (16) possède une liaison articulée.
